# EUROPÄISCHE PATENTSCHRIFT

(11) **EP 0 502 869 B1**
(45) Veröffentlichungstag und Bekanntmachung des Hinweises auf die Patenterteilung: **11.01.1995**
(21) Anmeldenummer: 90916739.7
(22) Anmeldetag: 19.11.1990
(51) Int. Cl.: A61L 25/00, A61K 6/08, C08F 2/40

(54) **VERBESSERTE REAKTIVSYSTEME UND/ODER POLYMERMASSEN FÜR DEN GEWEBEKONTAKT MIT DEM LEBENDEN KÖRPER**
IMPROVED REACTION SYSTEMS AND/OR POLYMER COMPOSITIONS FOR CONTACT WITH LIVING BODY TISSUE
SYSTEMES REACTIFS ET/OU MELANGES DE POLYMERES AMELIORES POUR LE CONTACT AVEC DES TISSUS D'ORGANISMES VIVANTS

(30) Priorität: 27.11.1989 DE 3939161
(43) Veröffentlichungstag der Anmeldung: 16.09.1992
(73) Patentinhaber: Henkel Kommanditgesellschaft auf Aktien, 40191 Düsseldorf (DE)
(72) Erfinder: RITTER, Wolfgang, D-5657 Haan (DE); SITZ, Hans-Dieter, D-4049 Rommerskirchen (DE)
(74) Vertreter: von Kreisler, Alek, Dipl.-Chem.
(86) Internationale Anmeldenummer: EP9001974
(87) Internationale Veröffentlichungsnummer: WO9107999

(56) Entgegenhaltungen:
- Deutsche Apothekerzeitung, vol. 128, No. 9, July 88 J. Lehmann:"Polymere Arzneistoffe und polymere Hilfsstoffe", S. 119, 132
- Chemical Abstracts, vl. 89, No. 14, 2 October 1978, (Columbus, Ohio, US), Enmanji, K. et al.: "The effects of alpha-tocopherol and its derivative on polymerisation of methyl methacrylate", p. 15, abstract 110589u
- Chemical Abstracts, vol. 103, No. 18, 4 November 1985, (Columbus, Ohio, US), Enmanji, K.: "The effect of alpha-tocopherol on polymerization of methyl methacrylate", p. 5, abstract 142453u
- Derwent's abstract, No. 86-295 159/45, JP 61 217 153, publ. week 8645

## Beschreibung

Die Erfindung betrifft Verbesserungen auf dem Gebiet der über Radikalmechanismen härtbaren Reaktivsysteme und/oder daraus gewonnenen Polymermassen, die mit dem lebenden Körper in Gewebekontakt gebracht werden und bei ihrer bestimmungsgemäßen Verwertung insbesondere in den lebenden menschlichen und/oder tierischen Organismus implantiert werden.

Sowohl in der Humanmedizin als auch im Bereich der Tiermedizin finden zunehmend Kunststoffe auf Polymerbasis sowie durch Reaktionsauslösung härtbare Reaktivsysteme Bedeutung. Verwiesen sei auf die chirurgischen und/oder zahnmedizinischen Klebstoffe, Zemente, Füllmassen und dergleichen, die gewöhnlich nach ihrer Applikation und Implantation in den lebenden Körper aushärten und dann im Kontakt mit dem lebenden Organismus verbleiben. Ebenso sind durch die Erfindung aber auch vorgebildete Polymermaterialien aus insbesondere radikalisch reaktiven Vorkomponenten betroffen, wobei diese Polymerkomponenten in ihrer praktischen Anwendung gezielt formgestaltet sein können oder aber auch lediglich als polymerer Mischungsbestandteil, beispielsweise eines reaktiven Klebstoffsystemes, in den lebenden Organismus eingetragen werden. Übereinstimmend gilt auch für diese unterschiedlichen Ausführungsformen, daß das vorgebildete Polymere in den lebenden Organismus implantiert wird und hier in aller Regel einem intensiven Kontakt mit dem Stoffwechsel des Körpers ausgesetzt ist.

Auf dem hier betroffenen Gebiet des Einsatzes von Kunststoffen bzw. Kunststoff-bildenden Reaktivsystemen in den lebenden Organismus lassen sich die beiden folgenden Hauptklassen voneinander unterscheiden: Nicht abbaubare körperresistente Kunststoffe, die für einen zeitlich begrenzten oder unbegrenzten Verbleib dem lebenden Organismus einverleibt werden, sowie die Klasse körperresorbierbarer Hilfsstoffe der genannten Art, die unter der Einwirkung des lebenden Stoffwechsels einem bevorzugt zeitgesteuerten Abbau unterliegen und gegebenenfalls schließlich vollständig resorbiert werden. Bestimmungsgemäß abbaubare Polymerkomponenten können beispielsweise chirurgische Klebstoffe für das Verkleben von körpereigenem Hartgewebe, insbesondere Knochen sein, bei denen die Bindefunktion des ausgehärteten Klebstoffes bestimmungsgemäß durch die Geweberegeneration - beispielsweise das Knochenwachstum - ersetzt wird. Andererseits wird von beispielsweise zahnmedizinischen Füllmassen und/oder Zementen das dauerhafte Verbleiben am Ort der Applikation gefordert. Gleiches gilt etwa für Zemente bzw. Kleber zum Einbinden von Implantaten - beispielsweise Hüftgelenksprothesen - in den menschlichen oder tierischen Organismus.

Es bestehen heute zahlreiche Vorschläge für den Einsatz von Kunststoffen und härtbaren Reaktivsystemen im und am lebenden Körper. Lediglich beispielhaft sei das an dem ausgewählten Einsatzgebiet der Bindemittelsysteme zum Verkleben von körpereigenem Hartgewebe, gewünschtenfalls auch mit Kunststoff und/oder Metall, geschildert.

Für die feste, dauerhafte Vereinigung von getrennten Knochenteilen oder die Fixierung von künstlichen Implantaten im bzw. am Knochen- oder Zahnmaterial sind bisher vorwiegend Polymethacrylate, Epoxidharz- und Polyurethansysteme untersucht worden. In der Praxis haben sich bisher überwiegend die Polymethacrylate durchgesetzt. Sie sind vorwiegend geeignet für die Implantation von Gelenkprothesen, die Zement-Metall-Ostheosynthese, Wirbelsäulenfusionen und Defektabdeckungen am Schädeldach bzw. Implantation von Stützmaterial in der Gesichtschirurgie. Die hier eingesetzten Zemente, Kleber und dergleichen sind üblicherweise nicht körperresorbierbar.

Neben diesen Anwendungen steht der Wunsch, zusätzliche Anwendungsfelder für Knochenklebstoffe bzw. Knochenzemente zu erschließen, beispielsweise im Zusammenhang mit einer anatomischen Reposition, Fixierung und Retention von Knochenfragmenten bei Trümmer- oder Gelenkbrüchen. Besonders wünschenswert erscheint hier die reversible Reposition, Fixierung und Retention von Knochenfragmenten mit Hilfe resorbierbarer Klebstoffsysteme. Hier soll der Knochenklebstoff bzw. -zement synchron zur Knochenneubildung vom Körper resorbiert werden. Weiterhin ist von Bedeutung die reversible Stabilisierung von Frakturen durch Platten oder Schienen, die mittels resorbierbarer Klebstoffe befestigt werden. Hier kann bei der Verwendung von Schienen oder Platten aus Metall bzw. Kunststoff auf das Anbringen von Bohrlöchern im Knochen und die Verwendung von Schrauben verzichtet werden. Bei Verwendung resorbierbarer Stützmaterialien z. B. aus Polymilchsäure oder Polyglykolsäure könnte zusätzlich die zweite Operation entfallen.

Die bisher in der Praxis eingesetzten härtbaren Klebstoffe, insbesondere auf Basis Methacrylatsystemen bestehen aus den folgenden Komponenten:
Ein oder mehrere radikalisch polymerisierbare Monomere, die in Abmischung mit Inhibitoren gegen eine unerwünschte vorzeitige radikalische Reaktionsauslösung vorliegen.

Ein Radikalstartersystem zur Polymerisationsauslösung, das nach Art und Menge ausreicht, die inhibierende Wirkung der zuvor genannten Stabilisatoren im Anwendungsfall zu überwinden.

Polymere zur Kohäsionsverbesserung und zur Einstellung der Viskosität sowie
gegebenenfalls aktive Füllstoffe zur Verbesserung der mechanischen Eigenschaften.

Als polymerisierbare Monomere sind neben Methylmethacrylat in Kombination mit Methacrylsäure zahlreiche weitere Systeme untersucht worden, von denen einige praktische Bedeutung gewonnen haben, vergleiche hierzu J. M. Antonucci, Polymer Science and Technology 14, 357 (1981). Auch auf der Seite der Härtersysteme für bei Raumtemperatur polymerisierende Methacrylatsysteme steht heute an sich eine breite Palette von Beschleunigern zur Verfügung - vgl. beispielsweise G. M. Brauer, Org. Coat. Plast. Chem. 42, 321 (1980) sowie J. W. Prane, Org. Coat. Plast. Chem. 40, 338 (1979).

In einer Mehrzahl von Vorschlägen der Anmelderin werden für die Polymerisationsauslösung hier betroffener radikalisch reaktiver Systeme Organoborverbindungen vorgeschlagen, vergleiche hierzu beispielsweise die DE-OSen 32 29 635 und 32 01 780 (jeweils A1). Die Erfindung geht von der Aufgabe aus, in gezielter Weise die physiologische Verträglichkeit solcher radikalisch reaktiver oder durch radikalische Polymerisation bzw. Härtung ausreagierter Systeme im Gewebskontakt mit dem lebenden Körper zu verbessern. Die Erfindung umfaßt dabei sowohl das Gebiet der nicht resorbierbaren Hilfsstoffe als das Gebiet der körperresorbierbaren Materialien.

In einer ersten Ausführungsform geht dabei die Erfindung von der folgenden Überlegung aus:
Es ist chemisches Grundwissen, daß bei Herstellung, Transport und/oder Lagerung der radikalisch reaktiven Systeme die Mitverwendung von Radikalinhibitoren zum sicheren Ausschluß einer unerwünscht frühzeitigen Abreaktion des Systems erforderlich ist. Die Praxis kennt hier den Zusatz zahlreicher Verbindungen bzw. Systeme. In Betracht kommen beispielsweise Hydride wie Lithiumaluminiumhydrid, Calciumhydrid oder Natriumborhydrid. Weitere bekannte Beispiele für diesen Zweck sind Phenole, Phenolderivate, Hydrochinon und Hydrochinonderivate oder insbesondere Phenothiazin. Als typische Beispiele seien etwa genannt Cumol, Hydrochinon, 2,6-Di-tert.-butyl-p-kresol, 2,6-Di-tert.-butyl-4-methoxyphenol, Bis(2-Hydroxy-3-tert.-butyl-5-methylphenyl)methan, Bis(3,5-di-tert.-butyl-4-hydroxyphenyl)methan, Bis(2-hydroxy-3-tert.-butyl-5-methylphenyl)sulfid, Bis(3-tert.-butyl-4-hydroxy-5-methylphenyl)sulfid oder auch Amine wie Diphenylamin, N,N'-Di-phenyl-p-phenylendiamin, 2-Phenylbenzimidazol, Anilin, Dinitrobenzol, 2-Nitro-alpha-naphthol, Tetraphenylethylen und Triphenylmethan.

Solche Radikalinhibitoren werden dabei in aller Regel nicht erst für die sichere Lagerung der Systeme bis zum Zeitpunkt der Applikation benötigt, schon bei der Herstellung der radikalisch reaktiven Verbindungen - beispielsweise der (Meth)-acrylsäureester - wird der Einsatz solcher Inhibitoren benötigt. Es läßt sich dementsprechend zwischen den sogenannten "Herstellungsinhibitoren" und den "Applikationsinhibitoren" unterscheiden, wobei diese beiden Typen der Inhibitoren nach Art und/oder Menge gleich oder ungleich sein können. Im ersten Fall ist der Applikationsinhibitor im allgemeinen der bereits bei der Herstellung eingesetzte Herstellungsinhibitor. Sehr häufig fordern jedoch die drastischen oder aus anderen Gründen schwierigen Herstellungsbedingungen der radikalisch reaktiven Komponenten den Einsatz vergleichsweise stark wirksamer Herstellungsinhibitoren, deren Mitverwendung bei der Applikation auf dem hier betroffenen Gebiet des Einsatzes am bzw. im lebenden Körper unerwünscht ist.

Die Erfindung setzt hier ein und geht dabei von der Konzeption aus, in dem mit dem lebenden Körper in Kontakt kommenden reaktiven oder ausreagierten Kunststoffmaterial einen ausgewählten Inhibitor optimaler Körperverträglichkeit zum Einsatz zu bringen. Der erfindungsgemäß ausgewählte Inhibitor sind physiologisch verträgliche Tocopherolverbindungen, insbesondere das Vitamin E.

Gegenstand der Erfindung ist dementsprechend in dieser ersten Ausführungsform die Verwendung von Vitamin E als Inhibitor gegen vorzeitige Polymerisationsauslösung in Reaktivsystemen, die zur radikalisch ausgelösten Polymerisation befähigt sind und vor und/oder nach ihrer Polymerisation mit dem lebenden Körper in Gewebekontakt gebracht und dabei insbesondere in den lebenden Organismus implantiert werden.

In der bevorzugten Ausführungsform wird Vitamin E als wenigstens überwiegender Bestandteil des Applikations-Inhibitorsystems von chirurgischen und/oder zahnmedizinischen Reaktivklebern und/oder -zementen bzw. entsprechenden Füllmassen eingesetzt. Diese bevorzugte Ausführungsform der Erfindung erfaßt weiterhin die Verwendung des Vitamin E als wenigstens überwiegenden Bestandteil des Applikations-Inhibitorsystems in Polymermassen, die vor ihrem Einbau in lebendes Gewebe hergestellt worden sind.

Die physiologische Bedeutung des Vitamin E im Körperstoffwechsel und insbesondere seine Schutzfunktion vor unerwünschtem radikalischen Angriff auf die lebende Zelle bzw. Zellbestandteile sind heute allgemein bekannt. Vitamin E wird dementsprechend beispielsweise in Kapselform in hohen Dosen, als Bestandteil von äußerlich applizierbaren Hautschutzmitteln wie Sonnenschutzcremes und dergleichen sowie als Zusatzstoff zu Nahrungsmitteln in breitem Umfang eingesetzt. Die Verträglichkeit des Vitamin E auch in hohen Dosen in seiner Interaktion mit dem lebenden Organismus ist gesichert. Die Erfindung sieht damit für den technischen Zweck der Inhibierung von Reaktivsystemen gegen vorzeitige Reaktionsauslösung den Einsatz eines Stoffes vor, der - im Gegensatz zur den zahlreichen anderen bekannten Inhibitoren für dieses Einsatzgebiet - optimale physiologische Verträglichkeit besitzt. Aber auch der Einsatz des Vitamin E in vorgebildeten Polymerverbindungen bringt einen vergleichbaren Vorteil. Auch die außerhalb des Körpers vorgebildeten Polymermassen werden bei ihrer Implantation in den menschlichen Körper einem intensiven Stoffaustausch mit dem lebenden Organismus unterworfen. Insbesondere in der Langzeitwirkung ist hier mit einem wenigstens partiellen Auswaschen des ursprünglich eingesetzten Inhibitorsystems und damit einer entsprechenden Belastung des lebenden Organismus zu rechnen. Im Sinne der erfindungsgemäßen Lehre ist dieser nicht zu unterbindende Prozeß praktisch problemlos.

Es kann erfindungsgemäß bevorzugt sein, das Vitamin E als alleinigen Applikationsinhibitor einzusetzen, wobei seine Verwendung in Mengen von etwa 200 bis 10000 ppm in Betracht kommen kann. Bevorzugt sind üblicherweise Vitamin E-Mengen im Bereich von etwa 300 bis 4000 ppm - jeweils bezogen auf das Gewicht des radikalisch reaktiven Stoffgemisches vor oder nach seiner Aushärtung. Reaktivmassen der hier betroffenen Art können dabei einkomponentige oder mehrkomponentige medizinische und/oder zahnmedizinische Klebstoffe, Zemente oder Füllmassen auf Basis üblicher monofunktioneller und/oder mehrfunktioneller olefinisch ungesättigter Verbindungen sein. Besondere Bedeutung kommt in diesem Zusammenhang der Acrylsäure und/oder der Methacrylsäure bzw. ihren Derivaten insbesondere mit einfunktionellen und/oder mehrfunktionellen Alkoholen zu, die im nachfolgenden der Einfachheit halber als (Meth)acrylate bezeichnet werden.

In einer wichtigen Ausführungsform der Erfindung ist das Vitamin E nicht nur als Applikationsinhibitor vorgesehen. Bevorzugt ist es hier, das Vitamin E auch schon als Herstellungsinhibitor bei der Synthese der reaktiven und/oder bereits abreagierten Bestandteile der mit dem lebenden Körper in Kontakt tretenden Massen einzusetzen. Hier wird zuverlässig das unerwünschte Übertragen von Inhibitor- bzw. Inhibitoranteilen aus der Herstellung der Reaktivkomponenten in das letztlich zum Einsatz kommende System und damit die Implantation unerwünschter Inhibitorbestandteile in den lebenden Organismus verhindert. Diese Variante der Erfindung wird allerdings erst dann besondere Bedeutung erhalten, wenn die Reinigung des radikalisch reaktiven Materials von seinem Herstellungsinhibitor Schwierigkeiten bereitet. Es sind in dem breiten Rahmen der hier betroffenen Hilfs- und Werkstoffe Ausführungsformen umfaßt, in denen eine solche Abtrennung des Herstellungsinhibitors durch konventionelle Verfahren, beispielsweise durch destillative Reinigung, möglich ist. In all diesen Fällen besteht in der Wahl des Herstellungsinhibitors für die Gewinnung des Reaktivsystems weitgehende Freiheit. Hier können beispielsweise besonders wirksame Inhibitorsysteme wie Phenothiazin, substituierte Hydrochinonverbindungen und dergleichen gewählt werden. Nach der Synthese beispielsweise eines monomeren (Meth)acrylates kann durch Destillation die Abtrennung der Reaktivverbindung vom schwerflüchtigen Inhibitor erfolgen. Das in dieser Weise gereinigte reaktive Gut wird dann mit Vitamin E im Sinne der Erfindung als Applikationsinhibitor stabilisiert und in dieser Form zum Einsatz gebracht.

Auf der anderen Seite kann gerade beispielsweise auf dem Gebiet der Klebstoffe die destillative Reinigung der Reaktivkomponenten und damit ihre Befreiung von einem ungeeigneten Herstellungsinhibitor schwer möglich oder unmöglich sein. Klebstoffsysteme, die beispielsweise wenigstens anteilsweise mehrfunktionelle hochsiedende Ester von (Meth)acrylsäure mit mehrfunktionellen Alkoholen enthalten, sind schon bei der Auswahl des Herstellungsinhibitors unter Berücksichtigung der erfindungsgemäßen Lehre auszugestalten. Eine destillative Abtrennung des Herstellungsinhibitors scheidet hier in aller Regel aus. Es kann sich dann der Einsatz von Vitamin E auch bereits in der Stufe der reaktiven schwerflüchtigen Verbindungen als Inhibitorzusatz anbieten.

In einer weiteren Ausführungsform der Erfindung ist allerdings auch bei Komponenten der zuletzt geschilderten Art ein Inhibitoraustausch möglich und vorgesehen wie er insbesondere in der parallelen Patentanmeldung ... (D 8929 "Verfahren zum Inhibitoraustausch in radikalisch reaktiven olefinisch ungesättigten Systemen") der Anmelderin beschrieben ist. Nach der Lehre dieses parallelen Schutzrechtes werden als Herstellungsinhibitoren entsprechende Verbindungen des Phenoltyps mit zur Salzbildung befähigten Hydroxylgruppen eingesetzt, die dann nach der Herstellung der Reaktivkomponenten durch Abreaktion mit festen basischen Komponenten, insbesondere festen Oxiden und/oder Hydroxiden der Erdalkalimetalle gebunden und aus dem Reaktionsgemisch entfernt werden. Im einzelnen wird auf die Angaben der genannten Parallelanmeldung verwiesen. Erfindungsgemäß wird dann der entfernte Herstellungsinhibitor bzw. der entsprechende Inhibitoranteil durch Vitamin E als Applikationsinhibitor ersetzt.

In einer bevorzugten weiteren Ausführungsform kommt das Vitamin E als Inhibitor in radikalisch reaktiven und/oder bereits ausreagierten Werkstoffen der geschilderten Art zum Einsatz, die frei sind von physiologisch bedenklichen Lösungsmitteln oder herstellungsbedingten Lösungsmittelresten. Auch hier kann wieder besondere Bedeutung solchen Komponenten zukommen, die selber nicht auf destillativem Wege als im Destillationsvorgang flüchtige Phase zu reinigen sind. Betroffen sind also auch hier insbesondere wieder mehrfunktionelle schwerflüchtige radikalisch reaktive Mischungsbestandteile, die beispielsweise durch Umsetzung von (Meth)acrylsäure mit mehrfunktionellen Hydroxylverbindungen erhalten werden. Systeme dieser zuletzt geschilderten Art sind auch schon bei ihrer Synthese stark vergelungsgefährdet. Sehr häufig wird nach dem Stand der Technik die Gefahr einer vorzeitigen Ab- bzw. Anreaktion dadurch gemindert, daß bei der Herstellung entsprechender mehrfunktioneller schwerflüchtiger Komponenten unter Einsatz von Lösungsmitteln gearbeitet wird, um durch den Verdünnungseffekt die Gefahr einer frühzeitigen Vergelung zu mindern. Die vergleichsweise leichtflüchtigen Lösungsmittel werden dann destillativ abgezogen, wobei häufig durch längere Behandlung im Hochvakuum versucht wird, auch letzte Losungsmittelreste abzutrennen. Es hat sich gezeigt, daß in aller Regel die wirklich vollständige Abtrennung der Losungsmittel schwierig ist, so daß hier eine zusätzliche Quelle einer unnötigen Belastung des lebenden Organismus bei der Implantation des Reaktivsystems bzw. eines daraus hergestellten Formteiles vorliegt. In der geschilderten bevorzugten Ausführungsform der Erfindung soll auch eine solche Belastung zuverlässig ausgeschaltet werden. Dementsprechend wird auf die Mitverwendung von insbesondere physiologisch bedenklichen Lösungsmitteln bei den Klebstoffen, Zementen, Füllmitteln und/oder entsprechenden Mischungskomponenten von vorneherein verzichtet.

Es leuchtet ein, daß durch die Vorschläge der Erfindung wichtige Verbesserungen für das besonders sensitive Gebiet der Kunststoffe bzw. Kunststoff-bildenden Reaktivsysteme in ihrer Interaktion mit dem lebenden Organismus geschaffen werden. Das gilt sowohl für Abbau-resistente Massen bzw. Formkörper der genannten Art wie insbesondere für Materialien, die vom lebenden Organismus abgebaut und dabei insbesondere zeitgesteuert resorbiert werden.

Gegenstand der Erfindung sind schließlich in einer weiteren Ausführungsform zum Verkleben von körpereigenem Gewebe geeignete chirurgische Bindemittelsysteme auf Basis einer radikalisch aktivierbaren Kleberkomponente, die in Abmischung mit einem Radikalinhibitor vorliegt, wobei die erfindungsgemäße Lehre dadurch gekennzeichnet ist, daß der Inhibitor oder wenigstens der überwiegende Inhibitoranteil Vitamin E ist. Insbesondere ist dieses Bindemittel außerdem frei von physiologisch bedenklichen Lösungsmitteln oder herstellungsbedingten Lösungsmittelresten.

Geeignet sind hier insbesondere konventionelle (Meth)acrylat-Reaktionsklebstoffe, die jetzt aber durch ihren Gehalt an Vitamin E als Inhibitor gekennzeichnet sind. Reaktionsklebstoffe dieser Art bestehen üblicherweise aus Methylmethacrylat, Acrylsäure, Polymethyimethacrylat (= Monomerharz) und einem Iniatorsystem. Als Knochenzement werden diese Klebstoffe in der Medizin zur Verankerung von Implantaten verwendet (J. Lehmann, Deutsche Apothekerzeitung, 128. Jahrg., Nr. 29, Seiten 119 ff.).

Erfindungsgemäß sind die eingesetzten Monomeren z. B. durch Destillation von den großtechnisch üblichen Herstellungsinhibitoren befreit und statt dessen mit Vitamin E inhibiert. In der bevorzugten Ausführungsform wird auch das als Mischungskomponente eingesetzte Polymethylmethacrylat aus einem Vitamin E als Inhibitor enthaltenden Monomeren gewonnen. Derart hergestellte Produkte sind weniger toxisch, die klebetechnischen Eigenschaften bleiben unverändert. Klebstoffharze dieser Art können sowohl durch konventionelle Redoxkatalyse - beispielsweise Peroxid/Amin-Systeme - als auch durch Einwirkung von UV-Licht in Gegenwart von UV-Initiatoren aber auch durch Einsatz von Boralkylverbindungen polymerisiert werden, wie sie beispielsweise beschrieben sind in den DE-OSen (jeweils A1) 30 41 843, 30 41 904, 31 43 945 und 32 01 780 der Anmelderin.

In den Rahmen der Erfindung fallen in dieser Ausführungsform insbesondere aber auch polyfunktionelle, bevorzugt in Abwesenheit von Lösungsmitteln hergestellte (Meth)acrylate, wie sie - und ihre Herstellung - in den älteren Patentanmeldungen P 38 43 854.2 (D 8492), P 38 43 938.7 (D 8493), P 38 43 930.1 (D 8494), P 38 43 843.7 (D 8483) beschrieben sind. Insbesondere wird dabei in der Patentanmeldung P 38 43 930.1 (D 8494) gleichzeitig auch schon die Verwendung von Vitamin E als Herstellungsinhibitor beschrieben. Die durch Abreaktion entstehenden Polymermassen sind hier in der Regel nicht körperresorbierbar.

Besonders geeignete Klebstoffkomponenten für den Einsatz im Rahmen der Erfindung sind allerdings Oligomerkomponenten, wie sie in den vorveröffentlichten DE-OSen (jeweils A1) 32 04 504 und 32 29 635 der Anmelderin beschrieben sind. Es handelt sich hierbei um Systeme, die als chirurgische Binde- bzw. Klebemittel für das Verkleben von körpereigenem Gewebe und/oder zur in situ Bildung von Kunststoff-Formteilen im Zuge chirurgischer Arbeiten in der Human- und Tiermedizin Verwendung finden können. Eine besondere Eigenart dieser Materialien liegt in ihrer Resorbierbarkeit durch körpereigene Abbaureaktionen, wobei gewünschtenfalls auch Einfluß auf die Zeitdauer des Resorptionsprozesses genommen werden kann. Bindemittelsysteme dieser Art zeichnen sich dadurch aus, daß sie wenigstens anteilig als resorbierbare (Meth)acrylatkomponente bei Raumtemperatur flüssige bis feste (Meth)acrylsäureester mit (Meth)acrylatresten an aus Hydroxycarbonsäuren gebildeten Polyester-Oligomerketten enthalten. Diese radikalisch reaktiven Komponenten können dabei einen oder bevorzugt auch mehrere (Meth)acrylsäurereste an dem Polyester-Oligomersegment aufweisen. Geeignet können insbesondere Reaktivkomponenten mit 2 bis 4 (Meth)acrylatresten an den Oligomersegmenten sein, die gewünschtenfalls in Abmischung mit nur einfach olefinisch ungesättigten (Meth)acrylatkomponenten zum Einsatz kommen.

Klebstoffsysteme auf Basis solcher Reaktivkomponenten zeichnen sich durch eine Mehrzahl wünschenswerter Eigenschaften aus. Sie besitzen aufgrund ihres niedrigen Dampfdrucks eine geringe Flüchtigkeit. Trotz vergleichsweise hohem Molekukargewicht besteht die Möglichkeit, sie bei Raumtemperatur als Flüssigkeiten oder wenigstens viskos streichbare Pasten auszubilden. Sie besitzen gute Mischbarkeit mit den üblicherweise eingesetzten weiteren Komponenten der hier betroffenen Bindemittelsysteme. Es lassen sich mit ihnen hoch feste, elastische Klebeverbindungen erzielen. Insbesondere führen diese ausgewählten reaktiven Monomeren zu ausgehärteten Kunststoffen, die vom lebenden Organismus resorbiert werden können. Ihr Einsatz im Rahmen chirurgischer Arbeiten führt damit zur reversiblen Ausbildung von Verklebungen, Stütz- bzw. Halterungselementen und dergleichen, die beispielsweise bei Knochenfrakturen eine zeitlich begrenzte Wirkung entfalten, letztlich aber wieder resorptiv abgebaut werden.

In den für die praktische Anwendung besonders wichtigen Verbindungstypen mit 2 (Meth)acrylatresten im Molekül sitzen diese funktionsfähigen Gruppen bevorzugt derart endständig am Oligomer-Segment, daß die beiden Endeinheiten des Oligomer-Segments jeweils mit einer (Meth)acrylatgruppe substituiert sind (Alpha,-omega-Stellung).

Die Oligomer-Segmente weisen das Strukturmerkmal 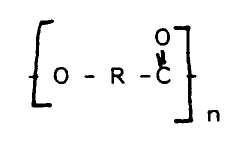
auf. Sie sind durch Oligomerisierung geeigneter Hydroxycarbonsäuren bzw. Hydroxycarbonsäuregemische der allgemeinen Formel 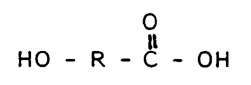
zugänglich. In einer bevorzugten Ausführungsform der Erfindung sind die Reste -R- und n derart ausgewählt bzw. aufeinander abgestimmt, daß das mittlere Molekulargewicht der Polyester-Oligomereinheit im Bereich von etwa 200 bis 600 liegt. Besonders bevorzugte Werte für das mittlere Molekulargewicht liegen im Bereich von etwa 300 bis 500.

Das Polyester-Oligomersegment wird in einer bevorzugten Ausführungsform aus Monohydroxymonocarbonsäuren gebildet, die eine C-Zahl von etwa 20 im Molekül und insbesondere von etwa 10 nicht überschreiten. Niedere Hydroxycarbonsäuren mit 2 bis 6 C-Atomen können in diesem Zusammenhang besonders wichtig sein. Besonders geeignete Hydroxycarbonsäuren zur Ausbildung dieses Mittelstücks der (Meth)acrylatverbindungen sind Glykolsäure, die isomeren Milchsäuren, die gegebenenfalls isomeren Alpha- oder Beta-Hydroxypropionsäuren, die gegebenenfalls isomeren Alpha-, Beta- oder Gamma-Hydroxybuttersäuren, o-Hydroxybenzoesäure (Salicylsäure) - m-Hydroxybenzoesäure und/oder p-Hydroxybenzoesäure (Anissäure). Es können dabei sowohl bestimmte Isomere der genannten Säuren als auch beliebige Gemische zum Einsatz kommen.

Die Polyester-Oligomeren sind dabei zweckmäßigerweise unter Mitverwendung von monofunktionellen und/oder vorzugsweise mehrfunktionellen Reaktanten hergestellt. Durch diese Co-Reaktanten erfolgt die Regelung des mittleren Molekulargewichts im Polyester-Oligomeren und damit die Einstellung des angestrebten Viskositätsbereiches. Zum anderen ist durch Auswahl der funktionellen Gruppen der mitverwendeten Co-Reaktanten die Möglichkeit gegeben, an den Polyester-Oligomeren einheitlich endständige Hydroxygruppen oder endständige Carboxylgruppen auszubilden. Schließlich gelingt es durch Mitverwendung ausgewählter monofunktioneller Co-Reaktanten eine reaktive Endgruppe der Polyester-Oligomeren zu beseitigen, so daß an diesem Typus von Verbindungen für die nachfolgende Anbindung der (Meth)acrylatreste nur eine reaktive Funktion zur Verfügung steht. Durch geeignete Abmischung von monofunktionellen und polyfunktionellen Co-Reaktanten ist es schließlich möglich, vorbestimmte Mischungsverhältnisse von Mono-(meth)-acrylatverbindungen und mehrfunktionellen (Meth)acrylatverbindungen einzustellen.

Als monofunktionelle Co-Reaktanten kommen insbesondere Monoalkohole und Monocarbonsäuren in Betracht. Polyfunktionelle Co-Reaktanten bei der Herstellung der Polyester-Oligomeren sind polyfunktionelle Alkohole, insbesondere 2- bis 4-wertige Alkohole oder entsprechende Polycarbonsäuren bzw. ihre funktionellen reaktiven Derivate. Co-Reaktanten mit funktionellen Hydroxylgruppen sind bevorzugt. Besondere Bedeutung kann hier niederen polyfunktionellen Alkoholen wie Ethylenglykol, Propandiol, insbesondere 1,2-Propandiol, Glycerin und dergleichen zukommen.

In allen Fällen entstehen modifizierte Polyester-Oligomere, die in an sich bekannter Weise einfach zu den reaktiven (Meth)acrylatverbindungen umgesetzt werden können. Liegen endständige Hydroxygruppen am Polyester-Oligomeren vor, so werden die (Meth)acrylsauregruppe(n) durch Veresterung bzw. Umesterung mit Acrylsäure oder Acrylsäureestern und/oder insbesondere entsprechenden Methacrylsäureverbindungen eingeführt.

In einer bevorzugten Ausführungsform der Erfindung werden (Meth)acrylatverbindungen bzw. entsprechende Gemische eingesetzt, die bei Raumtemperatur fließfähig oder wenigstens noch pastös verstreichbar sind, so daß sie als Hauptkomponente oder sogar als alleinige polymerisierbare Klebstoffkomponente in den Bindemitteln eingesetzt werden können. Es kann dabei bevorzugt sein, daß die (Meth)acrylatverbindungen bei Raumtemperatur eine Viskosität im Bereich von etwa 500 bis 70000 mPas, vorzugsweise im Bereich von etwa 3000 bis 50000 mPas besitzen. Feste (Meth)acrylatverbindungen der angegebenen Art lösen sich jedoch gut in flüssigen polymerisierbaren Komponenten, beispielsweise in Methylmethacrylat, so daß auch feste Verbindungen der angegebenen Konstitution in Abmischung mit flüssigen konventionellen Monomerbestandteilen in einfacher Weise zu den verarbeitungstechnisch gewünschten fließfähigen bzw. streichfähigen Klebstoffkomponenten aufgearbeitet werden können.

Die lösungsmittelfreie Herstellung von (Meth)acrylsäureestern mehrwertiger Alkohole unter Mitverwendung von Tocopherolen und insbesondere Vitamin E sowie die Reinigung der dabei anfallenden Reaktionsrohprodukte ist im einzelnen beschrieben in der älteren Patentanmeldung P 38 43 930.1 (D 8494 "Verfahren zur verbesserten Herstellung von (Meth)acrylsäureestern mehrwertiger Alkohole") der Anmelderin. Nach den Angaben dieses älteren Schutzrechtes arbeitet man mit bei Reaktionstemperatur flüssigen Reaktionsgemischen, die wenigstens weitgehend frei sind von Lösungs- und/oder azeotropen Schleppmitteln unter Einsatz von Vitamin E als Herstellungsinhibitor. Das bei der Veresterungsreaktion entstehende Kondensationswasser wird aus der Gasphase des Reaktionsraumes abgezogen. Bevorzugt wird dabei der Reaktionsraum mit einem freien Sauerstoff enthaltenden Gasstrom durchspült, wobei man Luft oder ein an O₂-angereichertes Gasgemisch, insbesondere entsprechende Stickstoff/Luft-Gemische einsetzen kann. Die Veresterung erfolgt bevorzugt bei Sumpftemperaturen von wenigstens etwa 90 °C, vorzugsweise von wenigstens etwa 100 °C und insbesondere im Bereich bis etwa 150 °C, wobei wenigstens absatzweise bei vermindertem, gegebenenfalls stufenweise zunehmend vermindertem Druck gearbeitet wird. Das als Herstellungsinhibitor hier eingesetzte Vitamin E wird zweckmäßigerweise in Mengen von 200 bis 10000 ppm, bevorzugt im Bereich von etwa 300 bis 4000 ppm - jeweils bezogen auf das Gewicht des Reaktionsgemisches - eingesetzt. Die Reaktion kann unter den angegebenen Lösungs- bzw. Schleppmittel-freien Bedingungen auf einen Umsatz von wenigstens 90 % der Theorie gebracht werden. Dabei werden - bevorzugt im Temperaturbereich von etwa 100 bis 140 °C - Reaktionszeiträume von etwa 10 Stunden, insbesondere von etwa 8 Stunden nicht oder nicht wesentlichen überschritten. Das Reaktionsrohprodukt kann einer trockenen Neutralisation - bevorzugt mit Oxiden und/oder Hydroxiden der Alkalimetalle, der Erdalkalimetalle und/oder des Aluminiums - unterworfen werden. Entsprechende Arbeitsbedinungen sind auch zur Herstellung der zuletzt erwähnten körperresorbierbaren mehrfunktionellen (Meth)acrylate geeignet.

Auch für Reaktivsysteme der hier zuletzt geschilderten Art sind zahlreiche Initiatorsysteme geeignet. In Betracht kommen insbesondere Redoxsysteme wie das bekannte System Dibenzoylperoxid/Dimethyltoluidin. Dabei eignen sich allerdings zur Polymerisationsauslosung vor allem auch wieder Boralkylverbindungen, wie sie in den zitierten vorveröffentlichten Schutzrechten der Anmelderin ausführlich beschrieben sind. Verwiesen wird in diesem Zusammenhang insbesondere nochmals auf die DE-OS (A1) 32 29 635, sowie auf die parallele Patentanmeldung ... (D 8943 "Neue Startersysteme für die Polymerisationsauslösung ethylenisch ungesättigter Systeme und ihre Verwendung").

In der zuletzt genannten parallelen Patentanmeldung der Anmelderin sind Startersysteme für die Polymerisationsauslösung ethylenisch ungesättigter Verbindungen auf Basis von Sauerstoffreaktiven Organo-Borverbindungen beschrieben, die dadurch gekennzeichnet sind, daß sie als Träger Oligoester von niederen Hydroxycarbonsäuren enthalten. Damit sind hier auch die Träger des Starter-Systems körperresorbierbare Komponenten. Geeignet sind als Träger insbesondere Oligoester von Hydroxycarbonsäuren mit 2 bis 10 C-Atomen, vorzugsweise mit 2 bis 5 C-Atomen, wobei besondere Bedeutung den Oligoestern der Glykolsäure und/oder Milchsäure zukommt. Geeignete Oligoester sind insbesondere niedrig-viskose Umsetzungsprodukte, die bevorzugt Viskositäten im Bereich bis etwa 40000 mPas, insbesondere bis maximal etwa 25000 mPas - bestimmt jeweils bei Raumtemperatur - aufweisen. Entsprechend niedrig-viskose Umsetzungsprodukte werden unter Mitverwendung von Alkoholen und/oder Carbonsäuren, vorzugsweise unter Einsatz mehrfunktioneller Alkohole hergestellt, wobei Umsetzungsprodukten aus Ethylenglykol und/oder Glycerin mit Glykolsäure und/oder Milchsäure besondere Bedeutung zukommen kann.

Schließlich ist aber auch der Einsatz von UV-Licht in Kombination mit UV-Initiatoren ein geeignetes Mittel zur Reaktionsauslösung.

Die Lehre der Erfindung erfaßt damit eine Vielzahl von Reaktiv-Systemen, die jeweils optimal an die Anforderungen des beabsichtigten Einsatzgebiets angepaßt werden können.

Die Polymer-bildenden Reaktiv-Komponenten können abbauresistent oder körperabbaubar sein. Gleiches gilt für gegebenenfalls mitverwendete vorgebildete Polymere. Auch Startersysteme, die eine Mitverwendung von oligomeren oder polymeren Trägern vorsehen, können wahlweise abbauresistent oder körperresorbierbar ausgebildet sein und in angepaßtersw Weise mit dem Gesamtsystem kombiniert werden.

Übereinstimmend gilt für alle Ausführungsformen der Erfindung, daß dem Vitamin E wenigstens als Applikationsinhibitor die überragende Bedeutung zukommt und daß in den bevorzugten Ausführungsformen die Massen frei von Lösungsmitteln bzw. Lösungsmittelresten sind.

Die nachfolgenden Beispiele beschreiben charakteristische Ausführungsformen der Erfindung für konventionelle nicht abbaubare Reaktivkleber sowie für körperresorbierbare Kleber der zuvor geschilderten Art.

### Beispiele

### Beispiel 1

### 1. Klebstoffharz

### a) Destillation der Monomeren Methacrylsäure und Methylmethacrylat

### Destillation der Methacrylsäure (zur Abtrennung von Hydrochinonmonomethylether) und direkte Neuinhibierung mit Vitamin E

In einer Vakuumdestillationsapparatur wurden 15 mol (1291,35 g) Methacrylsäure (Kp. 163 °C) mit 3,87 g (= 3000 ppm) Phenothiazin (als Stabilisator) versetzt und unter starkem Luftstrom im Wasserstrahlvakuum überdestilliert. In die Vorlage werden 100 ppm Vitamin E (Covitol F-1000-2, 67%ig, Henkel KGaA = 139 mg/l) vorgelegt und unter Rühren die Methacrylsäure überdestilliert. Die Destillation ist beendet, wenn von der Methacrylsäure 932 g überdestilliert sind.

### Destillation von Methylmethacrylat (zur Abtrennung von Hydrochinon) und direkte Neuinhibierung mit Vitamin E

Die Destillation erfolgt analog zur Destillation von Methacrylsäure.

### b) Herstellung der Klebstoffharze

### Allgemeine Vorschrift

In einem Becherglas wurden 40 g Polymethacrylsäuremethylester (PMMA) in 45 g Methacrylsäuremethylester (MMA) und 5 g Methacrylsäure (MAS), die wie unter 1 a) beschrieben destilliert wurden, unter Rühren gelöst.

### c) Klebeversuche

### mit Boralkylhärter

Zu 10 g dieser Mischung wurden unter weiterem intensivem Rühren 3 Gew.-% der unter 2 a) beschriebenen Boralkylstarter zugegeben. Die Topfzeit der Mischung betrug 4 Minuten. Mit diesen Klebstoffen wurden innerhalb der Topfzeit sandgestrahlte und entfettete Eisenbleche verklebt und nach 24 Stunden die Festigkeiten im Zugscherversuch nach DIN 53 281/3 gemessen. Die Ergebnisse sind in der Tabelle 1 zusammengestellt.

### mit Peroxid/Aminhärter des Klebstoffharzes

Zu 10 g des Monomerharzes nach 1 b) wurden unter intensivem Rühren zunächst 1 Gew.-% N,N-Dimethyl-toluidin und im Anschluß 2 Gew.-% Dibenzoylperoxid (als Lucidolpaste DBP 50 %) zugegeben und intensiv vermischt. Die Topfzeit der Mischung betrug 1,5 Minuten. Die Zugscherfestigkeiten sind ebenfalls in der Tabelle 1 aufgelistet.

### 2. Herstellung des Boralkylhärters

### Herstellung der Oligomeren

### Allgemeine Vorschrift zur Herstellung der Umsetzungsprodukte von Lactid mit Glycerin

Lactid (L(-)-Lactid N der Fa. Boehringer, Ingelheim) und Glycerin wurden in einer üblichen Laborapparatur unter Stickstoff und unter Rühren katalysiert mit 0,5 % Phsophorsäure - bezogen auf Lactid - innerhalb von einer Stunde auf 105 °C erwärmt. Man ließ dann 6 Stunden bei 195 °C reagieren und füllte heiß ab. Folgende Produkte wurden hergestellt:

| | | | |
|---|---|---|---|
| a) | Milchsäure: | Glycerin 3 : 1 | |
| | Ansatz: | 432 g Lactid | = 3 Mol |
| | | 184 g Glycerin | = 2 Mol |
| | Ausbeute: | 610 g/99 % | |
| b) | Milchsäure: | Glycerin 2 : 1 | |
| | Ansatz: | 1008 g Lactid | = 7 Mol |
| | | 644 g Glycerin | = 7 Mol |
| | Ausbeute: | 1621 g/98,2 % | |

Die Zusammensetzung der Produkte und ihre Eigenschaften sind in Tabelle 2 angegeben.

### Herstellung des Boroligomeradduktes

### Allgemeine Herstellvorschrift zur Herstellung des Boralkylhärters aus Milchsäureoligomeren nach 2 und 9-BBN

In einem Dreihalskolben mit Rührer und Thermometer wurden 100 g des Oligomeren nach 2 a) oder 2 b) vorgelegt. Nach Erwärmung auf ca. 50 °C wurde mit einer Drehschieberpumpe 3 x 10 Minuten auf 1 mbar evakuiert und jeweils mit Stickstoff belüftet.

Im Anschluß wurden im Stickstoffstrom die in der Tablle 3 angegebenen Mengen 9-Borabicyclo (3.3.1)nonan (9-BBN) zugegeben. Zur besseren Reaktionsführung wurden über einen Kolonnenkopf 100 ml THF (gelagert mit FeCl₂) in das Reaktionsgemisch destilliert. Das Gemisch wurde unter einem Stickstoffstrom 1,5 Stunden bei ca. 60 bis 65 °C gehalten. Danach wurde das THF mittels Wasserstrahlvakuum bei ca. 60 °C abdestilliert und die Restmengen THF bei 1 mbar abgezogen. Das Reaktionsprodukt wurde unter Stickstoffstrom in ein Vorratsgefäß überführt und unter N₂ verschlossen gelagert.

Die Herstellung der Härter und ihre Eigenschaften sind in Tabelle 3 zusammengestellt.

**Tabelle 1**

| Übersicht über die gemessenen Zugfestigkeiten/N/mm² | | | | | |
|---|---|---|---|---|---|
| Monomer-Klebstoffharz | Härterkomponente | | Topfzeit min. | Festigkeit in an Fe-Bleche nach 24 h RT/in N/mm² | Bor-Gehalt im Klebstoff/Gew.-% |
| | Bez. | Zusatz in Gew.-% | | | |
| 10 g | Bor-alkyl nach 2 a) | 3 | 4 | 27 | 0,11 |
| 10 g | Dimethyltoluidin | 1 | 1,5 | 25,6 | |
| | Dibenzoylperoxid | | | | |

**Tabelle 2**

| Oligohydroxycarbonsäuren aus Glycerin und Lactid | | | | |
|---|---|---|---|---|
| Beispiel | Glycerin | Lactid | Konsistenz bei Raumtemperatur | Viskosität Epprecht-Viskosimeter MK 4 bei Raumtemperatur |
| a | 1 | 1,5 | klar, leicht viskos | 18000 mPas |
| b | 1 | 1 | leicht gelb leicht, viskos | 16500 mPas |

**Tabelle 3**

| Darstellung der Initiatorkomponenten a 1, a 2, b | | | | |
|---|---|---|---|---|
| Nr. | Oligomeren Milchsäure : Glycerin | eingesetzte Menge 9-BBN/g pro 100 g Substanz | Produkteigenschaften | Gew.-% Bor |
| a 1 | 3 : 1 | 41 | homogen, farblos viskos | 3,64 |
| a 2 | 3 : 1 | 20 | homogen, farblos viskos | 1,78 |
| b | 2 : 1 | 41 | homogen, hellgelb, farblos | 3,64 |

### Beispiel 2

Eine körperresorbierbare und Vitamin E stabilisierte Klebstoffkomponente auf Basis Oligoglykolsäure-Bismethacrylat wird in den folgenden Verfahrensstufen hergestellt:
Zunächst wird aus Glykolsäure und Ethylenglykol im Molverhältnis 4 : 1 ein Hydroxylgruppen-terminiertes Oligomeres hergestellt. Dann wird handelsübliche Methacrylsäure unter Zusatz von Phenothiazin als Stabilisatorkomponente im Wasserstrahlvakuum destillativ von ihrem Inhibitorgehalt befreit. Die als Destillat übergegangene Methacrylsäure wird mit Vitamin E stabilisiert. Schließlich wird das Oligoglykolsäure-Vorkondensat mit der Methacrylsäure unter Zusatz von p-Toluolsulfonsäure als Katalysator und Zugabe einer weiteren Vitamin E-Menge im lösungsmittelfreien System verestert. Das Fortschreiten der Veresterungsreaktion wird kontrolliert. Erforderlichenfalls werden geringe zusätzliche Mengen an Methacrylsäure nachdosiert.

Das angefallene Reaktionsprodukt wird schließlich auf dem Wege der Trockenneutralisation mit Calciumhydroxid säurefrei gestellt und über einen Druckfilter von dem festen Neutralisationsmittel befreit.

Die eingesetzten Verfahrensschritte werden im nachfolgenden im einzelnen geschildert. Dabei wird mit einer Mehrzahl von Reaktionsansätzen gefahren. Das Alterungsverhalten der unter Luftausschluß gelagerten Oligoglykolsäure-Vorkondensate wird über den Zeitraum eines Jahres bestimmt. Dabei ist Konstanz der Produkteigenschaften festzustellen.

Im einzelnen gilt das folgende:

### 2.1 Herstellung des Oligomeren Glykolsäure/Ethylenglykol 4 : 1

In den 25-Liter-Versuchsreaktor wurden 16,72 kg Glykolsäure und 3,41 kg Ethylenglykol vorgelegt. Nach der Inertisierung wurde der Kristallbrei unter Stickstoff-Strom geschmolzen und dann weiter bis maximal 145 bis 150 °C (Sumpftemperatur) aufgeheizt. Nachdem die Reaktion durch Abdestillation von Wasser eingesetzt hatte, wurde sie 11 Stunden lang weitergeführt, bis sich kein Reaktionswasser mehr abspaltete (Brüdentemperaturabfall auf 70 bis 73 °C bei einem Reaktionsumsatz von 70 %). Von der abdestillierten wäßrigen Lösung wurde zur Quantifizierung die Menge des Destillats, die Säurezahl (Gehalt an Glykolsäure) und der Wassergehalt nach Karl-Fischer bestimmt. Um die Reaktion vollständig durchzuführen, wurde bei 150 °C vorsichtig auf 400 Torr evakuiert und innerhalb von 2 Stunden weiter bis auf 10 Torr reduziert und 1 Stunde gehalten, damit das restliche Reaktionswasser zur Quantifizierung abgezogen wurde.

Überschüssiges Kondensat wurde in einer Kühlfalle (gekühlt mit Trockeneis und Ethanol) aufgefangen und im Anschluß ebenfalls quantifiziert. Nach der gesamten Reaktionszeit wird auf 100 °C abgekühlt, das Vakuum mit Stickstoff aufgehoben und das Produkt noch heiß abgefüllt. Das Produkt wurde nicht weiter gereinigt und direkt zur Herstellung von Oligoglykolsäure-bis-methacrylat eingesetzt.

### 2.2 Ausbeute und Massenbilanz

| | |
|---|---|
| Menge Destillat | 4222,8 g |
| Glykolsäure aus SZ = 46 = 263,58 g | - 263,6 g |
| Ethylenglykolbestimmung aus HPLC | - |
| Wassermenge (aus Wasserbestimmung nach Karl-Fischer) = 91,6 % im Destillat = 3868,1 g = 97,68 % Umsatz | |

| Analysenergebnisse vom Oligomeren | | | |
|---|---|---|---|
| Bezeichnung | direkt nach Herstellung | 1 Monat alt | 1 Jahr alt |
| Ansatzgröße | 20 kg | 4,5 kg | 2 kg |
| Konsistenz | pastös | pastös | pastös |
| Viskosität/b. RT (Epprecht-Viskosimeter MK4) | 12500 mPas | 13000 mPas | 12800 mPas |

| Molgewicht: | | | |
|---|---|---|---|
| Mn* | 438 | 455 | 454 |
| Mw | 515 | 533 | 530 |
| %-freie Glykolsäure | 2,1/2,2 % | 1,4 % | 1,9 % |
| %-freies Ethylenglykol | 0,2 % | 0,2 % | 0,2 % |
| Verseifungszahl | 765,4 | 754,4 | 754,0 |

| Verhalten in Wasser | | | |
|---|---|---|---|
| pH-Wert nach 2 min. | 3,8 | 3,8 | 3,8 |
| pH-Wert nach 60 min. | 3,4 | 3,4 | 3,4 |
| Peroxid-Gehalt | negativ | | |

| analytische Zusammensetzung des Produktes | | | |
|---|---|---|---|
| Glykolsäure/Ethylenglykol | 3,975 : 1 | 3,972 : 1 | 3,972 : 1 |

| | | | |
|---|---|---|---|
| * Bestimmung des Molgewichtes als GPC-Analyse. Da die Eichung mit Polyethylenglykol als Standard durchgeführt wurde, erklärt sich die Differenz zwischen Mn theor. und Mn lt. Molgewichtsbestimmung durch die Eichmethode. | | | |

### 2.3 Oligoglykolsäure-bismethacrylat

Handelsübliche Methacrylsäure (Fa. Roehm) wird nach der folgenden Vorschrift neu mit Vitamin E inhibiert:
In einer Vakuumdestillationsapparatur werden 15 mol (= 1291,35 g) Methacrylsäure (kp. 163 °C mit 3,87 g (= 3000 ppm) Phenothiazin (als Stabilisator) versetzt und unter starkem Luftstrom im Wasserstrahlvakuum überdestilliert. In die Vorlage werden 100 ppm Vitamin E (Covitol F-1000-2, 67%ig, Henkel KGaA) (= 139 mg/l) vorgelegt und unter Rühren die Methacrylsäure überdestilliert. Die Destillation ist beendet, wenn von der Methacrylsäure 932 g überdestilliert sind.

### 2.4 Reaktionsverlauf

In einem Dreihalskolben mit Rührer und Destillationsbrücke wurden 294 g Oligoglkolsäure, 206,4 g Methacrylsäure sowie 17,5 g p-Toluolsulfonsäure eingewogen und mit 0,86 g Vitamin E (Alpha-Tocopherol) inhibiert. Während der gesamten Reaktion wurde Luft mit einer Geschwindigkeit von mindestens 40 l/h eingeleitet.

Durch Abtrennung des entstandenen Wassers bei einer Maximaltemperatur von 105 °C wurde die Veresterung durchgeführt bis die Menge an abgetrenntem Wasser über 35,78 g (mehr als 97 % Umsatz) liegt.

Die Destillationsvorlage wurde während der gesamten Reaktionsführung mit Trockeneis/Ethanolgemisch gekühlt. Bei einer maxmalen Sumpftemperatur von 105 °C und bei 500 mbar betrug die Veresterungszeit zwischen 12 und 14,5 Stunden bei einem Gesamtumsatz von 97 bis 98,5 %. Von der abdestillierten wäßrigen Lösung (aus Kühlfalle und Vorlage) wurde zur Quantifizierung alle 1,5 Stunden die Menge des Destillats, die Säurezahl (Gehalt an Methacrylsäure) und der Wassergehalt nach Karl-Fischer bestimmt.

Über eine Differenzberechnung wird der Wassergehalt und die überdestillierte Menge an Methacrylsäure berechnet, wobei nach jeder Bestimmung überprüft wurde, ob noch genügend Methacrylsäure für die Reaktion zur Verfügung stand. Meistens mußte nach ca. 7,5 bis 8 Stunden noch zusätzlich 0,1 mol Methacrylsäure nachdosiert werden.

Nach beendeter Reaktion (Umsatz über 97 %) wurde das Produkt bis zur Reinigung abgefüllt.

### 2.5 Aufarbeitung des Reaktionsproduktes

Das Reaktionsprodukt ist zum Ende der Reaktionszeit nicht ganz säurefrei. Deshalb wurde es mit Ca(OH)₂ neutralisiert. Da bei einer Säurezahlbestimmung durch das bei der Bestimmung benötigte Wasser sofortige Hydrolyse des Produktes stattfand und durch diese Reaktion immer mehr Säure frei wurde, war eine Titrationsbestimmung des Säuregehaltes nicht möglich.

Die Säurezahl mußte deshalb theoretisch berechnet werden.

Zur Neutralisation wurde die errechnete Menge an Ca(OH)₂ in das warme Reaktionsprodukt eingetragen und unter Rühren bei 105 °C, Durchleitung von 40 l Luft/h und 500 mbar für 30 Minuten behandelt.

Das neutralisierte Produkt (bei 100 bis 105 °C hochviskos) wird mit Hilfe einer Druckfilternutsche und einem Loeffler-Filter (80 NM012) bei 100 bis 105 °C unter 3 bar filtriert.

Anschließend wurde das noch heiße Produkt unter ansonsten gleichen Bedingungen über ein Rundfilter (NNG 16 mittelschnell filtrierend) nochmals filtriert.

### Beispiel 3

In einem 25-Liter-Glasreaktor mit Destillationsbrücke und Vorlage wurden 15 kg Acrylsäure inhibiert mit ca. 200 ppm Hydrochinonmonomethylether und 30 g Alpha-Tocopherol (Covitol F-1490 der Fa. Henkel KGaA) vorgelegt. Unter Durchleiten von 150 l Luft/h erfolgt die Destillation der Acrylsäure bei 50 mbar und 55 °C. Nach der Entnahme von ca. 3 l Vorlauf wurde die Hauptfraktion (7 kg) auf 3,6 g Alpha-Tocopherol (Covitol F-1490) destilliert, so daß die destillierte Acrylsäure mit ca. 500 ppm Alpha-Tocopherol inhibiert war.

| Kennzahlen: | |
|---|---|
| Säurezahl: | 778 mg KOH/g |
| Gardner Farbzahl: | kleiner 1 |
| Inhibitorgehalt: | 490 ppm Alpha-Tocopherol |

### Beispiel 4

In einem 2-Liter-Glasreaktor mit Destillationsbrücke, Vorlage und Rührer wurden 800 g eines ethoxylierten Trimethylolpropans (OH-Zahl 680 mg KOH/g Substanz), 839,1 g Acrylsäure (inhibiert mit 490 ppm Alpha-Tocopherol), 59,4 g p-Toluolsulfonsäure und 2,39 g Alpha-Tocopherol (Convitol F-1490 der Fa. Henkel KGaA; Gesamt-Inhibierung: 2000 ppm Alpha-Tocopherol bezogen auf Produktmenge) eingewogen.

Unter Durchleiten von Luft (40 l/h) wurde die Veresterung unter Wasser- und Acrylsäureüberschuß-Abtrennung durchgeführt. Bei einer Sumpftemperatur von 102 bis 103 °C betrug die Veresterungszeit 8 Stunden, dabei wurde folgendes Vakuumprofil eingehalten:
0 - 60 min. Druckreduzierung auf 500 mbar
60 - 300 min. 500 mbar
300 - 420 min. Druckreduzierung auf 50 mbar
420 - 480 min. 50 mbar

| Rohproduktkennzahlen: | |
|---|---|
| Säurezahl: | 20 mg KOH/g |
| OH-Zahl: | 12 mg KOH/g |
| Umsatz: | 96,9 % |
| Gardner Farbzahl: | 11 - 12 |
| H₂O-Gehalt: | 0,26 % |

Das Rohprodukt wurde durch Zugabe von 37,5 g festem Ca(OH)₂ und 1stündigem Rühren unter Durchleiten von Luft (40 l/h) und Wasserabtrennung bei 80 °C und 50 mbar neutralisiert.

| Produktdaten: | |
|---|---|
| Säurezahl: | kleiner 1 mg KOH/g |
| Hydroxylzahl: | 18 mg KOH/g |
| Gardner Farbzahl: | 3 - 4 |
| H₂O-Gehalt: | 0,10 % |
| Alpha-Tocopherolgehalt: | 810 ppm |

### Beispiel 5

In einem 2-Liter-Glasreaktor mit Destillationsbrücke, Vorlage und Rührer wurden 900 g eines propoxylierten Neopentylgkykols (OH-Zahl 460 mg KOH/g Substanz), 638,1 g Acrylsäure (inhibiert mit 490 ppm Alpha-Tocopherol), 55,8 g p-Toluolsulfonsäure und 2,29 g Alpha-Tocopherol (Convitol F-1490 der Fa. Henkel KGaA; Gesamt-Inhibierung: 2000 ppm Alpha-Tocopherol bezogen auf Produktmenge) eingewogen.

Unter Durchleiten von Luft (40 l/h) wurde die Veresterung unter Wasser- und Acrylsäureüberschuß-Abtrennung durchgeführt. Bei einer Sumpftemperatur von 102 bis 103 °C betrug die Veresterungszeit 8 Stunden, dabei wurde folgendes Vakuumprofil eingehalten:
0 - 60 min. Druckreduzierung auf 500 mbar
60 - 300 min. 500 mbar
300 - 420 min. Druckreduzierung auf 50 mbar
420 - 480 min. 50 mbar

| Rohproduktkennzahlen: | |
|---|---|
| Säurezahl: | 25 mg KOH/g |
| Hydroxylzahl: | 10 mg KOH/g |
| Umsatz: | 96,6 % |
| Gardner Farbzahl: | 8 |
| H₂O-Gehalt: | 0,19 % |

Das Rohprodukt wurde durch Zugabe von 46,3 g festem Ca(OH)₂ und 1stündigem Rühren unter Durchleiten von Luft (40 l/h) und Wasserabtrennung bei 80 °C und 50 mbar neutralisiert.

| Produktdaten: | |
|---|---|
| Säurezahl: | kleiner 1 mg KOH/g |
| Hydroxylzahl: | 16 mg KOH/g |
| Gardner Farbzahl: | 3 |
| H₂O-Gehalt: | 0,11% |
| Alpha-Tocopherolgehalt: | 630 ppm |

## Patentansprüche

1. Verwendung von Vitamin E als Inhibitor gegen vorzeitige Polymerisationsauslösung in Reaktivsystemen, die zur radikalisch ausgelosten Polymerisation befähigt sind und vor und/oder nach ihrer Polymerisation mit dem lebenden Körper in Gewebekontakt gebracht und dabei insbesondere in den lebenden Organismus implantiert werden.

2. Ausführungsform nach Anspruch 1, dadurch gekennzeichnet, daß Vitamin E als wenigstens überwiegender Bestandteil des Applikations-Inhibitorsystems von chirurgischen und/oder zahnmedizinischen Füllmassen, Reaktivklebern und/oder -zementen eingesetzt wird und/oder in entsprechender Weise in Polymermassen vorliegt, die vor ihrem Einbau in lebendes Gewebe hergestellt worden sind.

3. Ausführungsform nach Ansprüchen 1 und 2, dadurch gekennzeichnet, daß Vitamin E als alleiniger Applikationsinhibitor eingesetzt und dabei in Mengen von 200 bis 10000 ppm, bevorzugt in Mengen im Bereich von etwa 300 bis 4000 ppm - jeweils bezogen auf das Gewicht des Reaktionsgemisches - verwendet wird.

4. Ausführungsform nach Ansprüchen 1 bis 3, dadurch gekennzeichnet, daß Vitamin E als Inhibitor in 1- und/oder mehrkomponentigen medizinischen und/oder zahnmedizinischen Klebstoffen, Zementen oder Füllmassen auf Basis monofunktioneller und/oder mehrfunktioneller (Meth)acrylate eingesetzt wird.

5. Ausführungsform nach Ansprüchen 1 bis 4, dadurch gekennzeichnet, daß der Inhibitor auf Vitamin E-Basis in abbauresistenten Massen der geschilderten Art eingesetzt wird oder aber in solchen Massen bzw. Formkörpern vorliegt, die vom lebenden Organismus abgebaut, insbesondere zeitgesteuert resorbiert werden.

6. Ausführungsform nach Ansprüchen 1 bis 5, dadurch gekennzeichnet, daß Vitamin E auch als Herstellungsinhibitor bei der Synthese der reaktiven und/oder bereits abreagierten Bestandteile der mit dem lebenden Körper in Kontakt tretenden Massen eingesetzt worden ist.

7. Ausführungsform nach Ansprüchen 1 bis 6, dadurch gekennzeichnet, daß das Vitamin E in radikalisch reaktiven und/oder bereits ausreagierten Werkstoffen zum Einsatz kommt bzw. vorliegt, die frei sind von physiologisch bedenklichen Lösungsmitteln oder herstellungsbedingten Lösungsmittelresten und insbesondere in lösungsmittelfreien Syntheseverfahren hergestellt worden sind.

8. Zum Verkleben von körpereigenem Gewebe geeignetes chirurgisches Bindemittelsystem auf Basis einer radikalisch aktivierbaren Kleberkomponente, die in Abmischung mit einem Radikalinhibitor vorliegt, dadurch gekennzeichnet, daß der Inhibitor oder wenigstens der überwiegende Inhibitoranteil Vitamin E ist.

9. Bindemittel nach Anspruch 8, dadurch gekennzeichnet, daß es frei ist von physiologisch bedenklichen Lösungsmitteln oder herstellungsbedingten Lösungsmittelresten.

10. Bindemittel nach Ansprüchen 8 und 9, dadurch gekennzeichnet, daß es in der Kleberkomponente härtbare und/oder polymerisierbare (Meth)acrylsäureester enthält, die auch in Abmischung mit vorgebildeten Polymerverbindungen vorliegen können, wobei die (Meth)acrylsäureester sowie die durch Härtung daraus entstehenden und/oder die vorgebildeten Polymerverbindungen abbauresistent oder aber auch körperresorbierbar ausgebildet sein können.

11. Bindemittel nach Ansprüchen 8 bis 10, dadurch gekennzeichnet, daß sie als körperresorbierbare (Meth)acrylatkomponenten bei Raumtemperatur flüssige bis feste (Meth)acrylsäureester mit (Meth)acrylatresten an Polyester-oligomerketten aus Hydroxycarbonsäuren, insbesondere niederen Hydroxycarbonsäuren mit 2 bis 6 C-Atomen enthalten.

12. Bindemittel nach Ansprüchen 8 bis 11, dadurch gekennzeichnet, daß die resorbierbare (Meth)acrylatkomponente wenigstens anteilsweise mehr als einen (Meth)acrylatrest - vorzugsweise 2 bis 4 (Meth)acrylatreste - an den Hydroxycarbonsäure-polyesteroligomeren aufweist.

13. Bindemittel nach Ansprüchen 8 bis 12, dadurch gekennzeichnet, daß die (Meth)acrylatreste wenigstens anteilsweise endständig an den Polyesteroligomeren vorliegen, wobei bevorzugt 2 (Meth)acrylatreste in Alpha-, Omega-Stellung vorgesehen sind.

14. Bindemittel nach Ansprüchen 8 bis 13, dadurch gekennzeichnet, daß die mono- und/oder polyfunktionellen (Meth)acrylsäureester auf Polyesteroligomeren der Glycolsäure, Milchsäure, Hydroxypropionsäure und/oder Hydroxybuttersäure basieren.

15. Bindemittel nach Ansprüchen 8 bis 14, dadurch gekennzeichnet, daß die körperresorbierbaren (Meth)acrylatester sich von Polyesteroligomeren ableiten, die unter Mitverwendung von monofunktionellen und/oder mehrfunktionellen Alkoholen oder Carbonsäuren hergestellt worden sind, wobei entsprechende Polyesteroligomere der Glycolsäure und/oder der Milchsäure, die unter Mitverwendung von niederen difunktionellen und/oder trifunktionellen Alkoholen hergestellt worden sind, besonders bevorzugt sein können.

16. Bindemittel nach Ansprüchen 8 bis 15, dadurch gekennzeichnet, daß die härtbaren (Meth)acrylatester auf Polyester-Oligomeren eines mittleren Molekulargewichtes im Bereich von etwa 200 bis 600, vorzugsweise im Bereich von etwa 300 bis 500 basieren.

17. Bindemittel nach Ansprüchen 8 bis 16, dadurch gekennzeichnet, daß die Reaktiv-Massen insbesondere auf Basis abbauresistenter oder körperresorbierbarer (Meth)acrylatkomponenten bei Raumtemperatur fließfähig bis pastös verstreichbar sind und dabei bevorzugt bei Raumtemperatur eine Viskosität im Bereich von etwa 500 bis 70000 mPas aufweisen.

## Claims

1. Use of vitamin E as an inhibitor against premature polymerization initiation in reactive systems capable of undergoing a free radical-initiated polymerization, which systems before and/or after the polymerization thereof are placed in tissue contact with the living body and, more specifically, are implanted into the living organism thereby.

2. An embodiment according to claim 1, characterized in that vitamin E is employed as the at least predominant constituent of the application inhibitor system of surgical and/or dental-medical fillers, reactive adhesives and/or cements and/or is present in a corresponding manner in polymer compositions which have been prepared prior to the incorporation thereof in living tissue.

3. An embodiment according to claims 1 and 2, characterized in that vitamin E is employed as the only application inhibitor and is used in amounts of from 200 to 10,000 ppm, and preferably in amounts ranging from about 300 to about 4,000 ppm - each based on the weight of the reaction mixture.

4. An embodiment according to claims 1 to 3, characterized in that vitamin E is employed as inhibitor in mono-component and/or multi-component medical and/or dental-medical adhesives, cements or fillers based on conventional monofunctional and/or polyfunctional (meth)acrylates.

5. An embodiment according to claims 1 to 4, characterized in that the vitamin E-based inhibitor is employed in degradation-resistant compositions of the kind described or is present in compositions or molded bodies which are decomposed by the living organism, and especially are resorbed under time-controlled conditions.

6. An embodiment according to claims 1 to 5, characterized in that vitamin E has also been employed in the synthesis of the reactive and/or already reacted constituents of the compositions getting into contact with the living body.

7. An embodiment according to claims 1 to 6, characterized in that the vitamin E is used or is present in materials which are reactive via free radicals and/or which have already completed the reaction, said materials being free from physiologically unacceptable solvents or residual amounts of solvents left from the preparation process and, more particularly, having been prepared in synthesis processes conducted in the absence of solvents.

8. Surgical binder system which is suitable for adhesion bonding endogenous body tissue and which has been based on an adhesive component capable of being activated by free radicals, said adhesive component being present in admixture with a free radical inhibitor, characterized in that the inhibitor or at least the predominant portion of the inhibitor is vitamin E.

9. The binder according to claim 8, characterized in that it is free from physiologically unacceptable solvents or residual amounts of solvents left from the preparation process.

10. The binder according to claims 8 and 9, characterized in that it contains, in the adhesive component, curable and/or polymerizable (meth)acrylic acid esters, which may also be present in admixture with previously formed polymer compounds, wherein the (meth)acrylic acid esters as well as polymer compounds formed therefrom by curing and/or the previously formed polymer compounds may be formed to be degradation-resistant or resorbable by the body.

11. The binders according to claims 8 to 10, characterized in that they contain, as the body-resorbable (meth)acrylate component, (meth)acrylic acid esters which are liquid to solid at room temperature and have (meth)acrylate moieties on polyester-oligomer chains of hydroxycarboxylic acids, and especially of lower hydroxycarboxylic acids having from 2 to 6 carbon atoms.

12. The binders according to claims 8 to 11, characterized in that the body-resorbable (meth)acrylate component at least in part comprises more than one (meth)acrylate moiety - and preferably from 2 to 4 (meth)acrylate moieties - on the hydroxycarboxylic acid polyester-oligomers.

13. The binders according to claims 8 to 12, characterized in that the (meth)acrylate moieties at least in part are present in terminal positions on the polyester-oligomers, whereby it is preferred that two (meth)acrylate moieties have been provided in the α,ω-positions.

14. The binders according to claims 8 to 13, characterized in that the mono- and/or polyfunctional (meth)acrylic acid esters have been based on polyester-oligomers of glycolic acid, lactic acid, hydroxypropionic acid and/or hydroxybutyric acid.

15. The binders according to claims 8 to 14, characterized in that the body-resorbable (meth)acrylate esters are derived from polyester-oligomers which have been prepared with the concomitant use of monofunctional and/or polyfunctional alcohols or carboxylic acids, among which the respective polyester-oligomers of glycolic acid and/or of lactic acid prepared with the concomitant use of lower difunctional and/or trifunctional alcohols may be particularly preferred.

16. The binders according to claims 8 to 15, characterized in that the curable (meth)acrylate esters have been based on polyester-oligomers having an average molecular weight within the range of from about 200 to 600, and preferably within the range of from about 300 to 500.

17. The binders according to claims 8 to 16, characterized in that the reactive compositions, especially those based on degradation-resistant or body-resorbable (meth)acrylate components, are fluid to pastous spreadable and preferably have a viscosity at room temperature within the range of from about 500 to 70,000 mPa·s.

## Revendications

1. Utilisation de la vitamine E comme inhibiteur du déclenchement prématuré de la polymérisation dans les systèmes réactifs qui sont susceptibles de subir la polymérisation déclenchée par voie radicalaire et qu'on met en contact avant et/ou après leur polymérisation avec le tissu du corps vivant et qu'on implante notamment dans l'organisme vivant.

2. Mode de réalisation selon la revendications 1, caractérisé en ce que la vitamine E se trouve au moins comme composant principal du système inhibiteur d'application dans des mastics chirurgicaux et/ou dentaires, des adhésifs réactifs et/ou des ciments réactifs et/ou elle se trouve de manière appropriée dans des masses de polymère qu'on a préparées avant leur implantation dans le tissu vivant.

3. Mode de réalisation selon les revendications 1 et 2, caractérisé en ce qu'on utilise la vitamine E comme inhibiteur d'application unique et ce en quantités comprises entre 200 et 10.000 ppm, de préférence en des quantités comprises entre environ 300 et 4000 ppm, chaque fois rapportées au poids du mélange réactionnel.

4. Mode de réalisation selon les revendications 1 à 3, caractérisé en ce qu'on utilise la vitamine E comme inhibiteur dans des adhésifs médicaux et/ou dentaires mono- et/ou polycomposants dans des ciments ou mastics à base de (méth)acrylates.

5. Mode de réalisation selon les revendications 1 à 4, caractérisé en ce que l'inhibiteur à base de vitamine E est utilisé dans des masses résistantes à la dégradation du type indiqué, mais aussi existe dans des masses ou des corps moulés, qui sont dégradés par l'organisme vivant, notamment qui sont résorbés dans le temps.

6. Mode de réalisation selon les revendications 1 à 5, caractérisé en ce qu'on utilise aussi la vitamine E comme inhibiteur de préparation lors de la synthèse des composants réactifs et/ou qui ont déjà réagi des masses entrant en contact avec le corps vivant.

7. Mode de réalisation selon les revendications 1 à 6, caractérisé en ce que la vitamine E se trouve ou est utilisée dans des matériaux réactifs par voie radicalaire et/ou qui ont déjà réagi, qui sont exempts de solvants nuisibles physiologiquement ou de restes de solvants provenant de la préparation et notamment qu'on a préparé dans des procédés de synthèse sans solvant.

8. Système de liant chirurgical approprié pour coller du tissu corporel, à base d'un composant d'adhésif activable par voie radicalaire, qui contient en mélange un inhibiteur de radicaux, caractérisé en ce que l'inhibiteur ou au moins la majorité de l'inhibiteur est de la vitamine E.

9. Liants selon la revendication 8, caractérisés en ce qu'il sont exempts de solvants physiologiquement nuisibles ou de restes de solvant résultant de la préparation.

10. Liants selon les revendications 8 et 9, caractérisés en ce qu'ils contiennent dans le composant d'adhésif des esters d'acide (méth)acrylique durcissables et/ou polymérisables, qui peuvent se trouver aussi en mélange avec des composés polymères préformés, les (méth)acrylates ainsi que les composés polymères résultant du durcissement et/ou les composés polymères préformés sont résistants à la dégradation, mais on peut les constituer aussi de façon à être résorbables dans le corps.

11. Liants selon les revendications 8 à 10, caractérisés en ce qu'ils contiennent comme composants de (méth)acrylate résorbables dans le corps des esters d'acide (méth)acrylique liquides à solides avec des restes de (méth)acrylates sur des chaînes oligomères polyesters à base d'acides hydroxycarboxyliques, notamment des acides hydroxycarboxyliques inférieurs avec de 2 à 6 atomes de C.

12. Liants selon les revendications 8 à 11, caractérisés en ce que le composant (méth)acrylate résorbable comprend au moins en partie plus d'un reste (méth)acrylate de préférence 2 à 4 restes (méth)acrylate sur les oligomères polyesters d'acide hydroxycarboxylique.

13. Liants selon les revendications 8 à 12, caractérisés en ce que les restes (méth)acrylates se trouvent au moins en partie aux extrémités des oligomères polyesters et de préférence on prévoit les 2 restes (méth)acrylates en position alpha et oméga.

14. Liants selon les revendications 8 à 13, caractérisés en ce que les esters d'acide (méth)acrylique mono et/ou polyfonctionnels sont à base d'oligomères polyesters de l'acide glycolique, de l'acide lactique, de l'acide hydroxypropionique et/ou de l'acide hydroxybutyrique.

15. Liants selon les revendications 8 à 14, caractérisés en ce que les esters (méth)acrylates résorbables dans le corps dérivent d'oligomères polyesters, qu'on prépare en présence d'alcools ou d'acides carboxyliques monofonctionnels et/ou polyfonctionnels, on préfère particulièrement les oligomères polyesters correspondants de l'acide glycolique et/ou de l'acide lactique, qu'on prépare par réaction avec des alcools difonctionnels et/ou trifonctionnels.

16. Liants selon les revendications 8 à 15, caractérisés en ce que les esters (méth)acrylates sont à base d'oligomères polyesters ayant un Poids moléculaire moyen compris entre environ 200 à 600, de préférence entre environ 300 à 500.

17. Liants selon les revendications 8 à 16, caractérisés en ce que les masses réactives notamment celles à base de composants (méth)acrylate résistants à la dégradation ou résorbables dans le corps peuvent couler à température ambiante ou sont étalables sous forme pâteuse et présentent de préférence à température ambiante une viscosité comprise entre environ 500 et 70.000 mPas.
